Europäisches Patentamt

**European Patent Office**

**Office européen des brevets**

(19)

(11) Publication number: **0 104 772**
**B1**

(12) # EUROPEAN PATENT SPECIFICATION

(45) Date of publication of patent specification: **21.03.90**

(51) Int. Cl.⁵: **A 61 B 5/00, A 61 B 5/02**

(21) Application number: **83304940.6**

(22) Date of filing: **25.08.83**

(60) **Divisional application 89105503.0 filed on 25/08/83.**

(54) Calibrated optical oximeter probe.

(30) Priority: **02.09.82 US 414176**

(43) Date of publication of application:
**04.04.84 Bulletin 84/14**

(45) Publication of the grant of the patent:
**21.03.90 Bulletin 90/12**

(84) Designated Contracting States:
**AT BE CH DE FR GB IT LI LU NL SE**

(56) References cited:
**DE-A-3 100 610**
**FR-A-1 589 461**
**US-A-3 910 701**
**US-A-4 086 915**
**US-A-4 266 554**

(73) Proprietor: **NELLCOR INCORPORATED**
**25495 Whitesell Street**
**Hayward California 94545 (US)**

(72) Inventor: **New, William, Jr.**
**95 Skywood Way**
**Woodside California (US)**
Inventor: **Corenman, James E.**
**708 Harbor Road**
**Alameda California (US)**

(74) Representative: **Vossius & Partner**
**Siebertstrasse 4 P.O. Box 86 07 67**
**D-8000 München 86 (DE)**

Courier Press, Leamington Spa, England.

## Description

This invention relates to a probe for an oximeter monitor and oximeter for the determination of arterial oxygen saturation and/or pulse rate in a patient.

A serious problem exists in operating rooms. Specifically, the chemical determination of oxygen level in blood consumes at least 3 to 5 minutes. A patient deprived of blood oxygen for such a duration typically incurs irreversible brain damage if not death.

U.S. Patent No. 2,706,927 to Wood disclosed the computation of oxygen saturation from measurements of light absorption of body tissue at two wavelengths. A series of devices and procedures have been founded using this technology.

A required peripheral device of such photoelectric oximeters is a photoelectric probe. Typically, such a probe is clamped to an appendage of a patient's body, such as an ear or a finger. Such probes require at least one light source for directing light into the appendage and at least one sensor for receiving light diffused out of the appendage. One method of obtaining light of the desired frequency has been to use a light surce of indeterminate wavelength range in combination with a monochromatic filter of known output. Such devices are inefficient, and result in unwanted power demands and heat generation.

U.S. Patent No. 3,704,706 to Herczfeld et al disclosed the use of a solid state red laser in an optical probe with a solid state photodetector. Although lasers are useful for emitting monochromatic light of known wavelength, thereby eliminating need for a filter, they remain expensive and unwieldy.

U.S. Patent No. 3,847,483 to Shaw et al. disclosed the use of light emitting diodes to provide the necessary monochromatic light. The probe of Shaw required expensive fiber optic cables.

A problem with all prior art devices is that they are too expensive to be readily disposable. The need for a truly disposable probe is great, given the many surgical applications in which sterility must be assured. The prior art optical probes, being more or less permanent portions of their respective oximeters, were subjected to a one time determination of the wavelength of the light sources therein and the oximetre was then programmed or adjusted to process light of the known wavelength.

A problem in developing disposable probes, therefore, has been the necessity to avoid having to reprogram or adjust the oximeter for each new probe or alternately to maintain probes within narrow limits of wavelength variation, a clearly impractical task.

Re-calibration, perhaps necessitating return of the oximeters to the factory, can become necessary even for prior art devices when, for example, a probe is broken. Alternatively, a supply of light sources having consistently identical wavelengths is required. In particular, light emitting diodes are known to vary in wavelengths from unit-to-unit.

Other optical probes are shown in patents to Shaw, U.S. Patent No. 3,638,640, Neilsen, U.S. Patent No. 4,167,331, and Konishi, U.S. Patent No. 3,998,550.

According to the invention, an oximeter probe for use with an oximeter is provided; said probe comprises a first light emitting means emitting light having a first known wavelength value; means for sensing the light emitted by said first light emitting means; means for detachably wiring the probe to the oximeter and for providing communication of electrical signals between the probe and the oximeter; and encoding means for providing signals to the oximeter which are indicative of the known wavelength value of said first light emitting means.

An advantage of the embodiment of the present invention is that the apparatus is inexpensive, replaceable, easily applied and overcomes the disadvantages and limitations of the prior art as detailed above.

The embodiment has a probe whose wavelength emission characteristics are readily ascertainable by the attendant oximeter.

This embodiment enables factory calibration of LEDs for use in such a probe. Typically, LEDs are purchased in batches of one general wavelength, but whose exact wavelength characteristics are unknown and vary from piece to piece.

It is a further feature of this embodiment that it eliminates the necessity for oximeters to be calibrated for new probes, other than the initial factory calibration.

Yet another feature of this embodiment is that it provides flexible attachment means for the probe which will allow rapid attachment to human or animal appendages of varying sizes yet maintain the photoelectric sensor in direct optical isolation from the LEDs.

Yet another feature of this embodiment is that it has a multiconductor plug with wiring in a binary array to transmit probe calibration.

Furthermore the invention provides an oximeter system comprising the above-mentioned probe and an oximeter; this system is characterized by decoding means responsive to said encoded signals for selecting appropriate calibration coefficients for use in calculating oxygen saturation based upon the known wavelength of said first and/or second light emitting means.

Embodiments of the present invention will now be described by way of example with reference to the accompanying drawings in which:—

Fig. 1 is a part perspective, part schematic diagram of the optical probe of the preferred embodiment of the present invention.

Fig. 2 is an end view of a patient's finger showing implacement of the probe of the present invention.

Fig. 3 is a side elevation of an embodiment of a photoelectric sensor of the probe of Fig. 2.

Fig. 4 is a simplified schematic circuit diagram illustrating the method in which an oximeter microprocessor decodes the wavelength values of the probe through use of a coded resistor.

Fig. 5 is a schematic of the probe of this invention calibrated by a multiconductor plug and wired in a binary array and

Fig. 6 is a circuit schematic of an oximeter utilizing a calibrated probe of this invention.

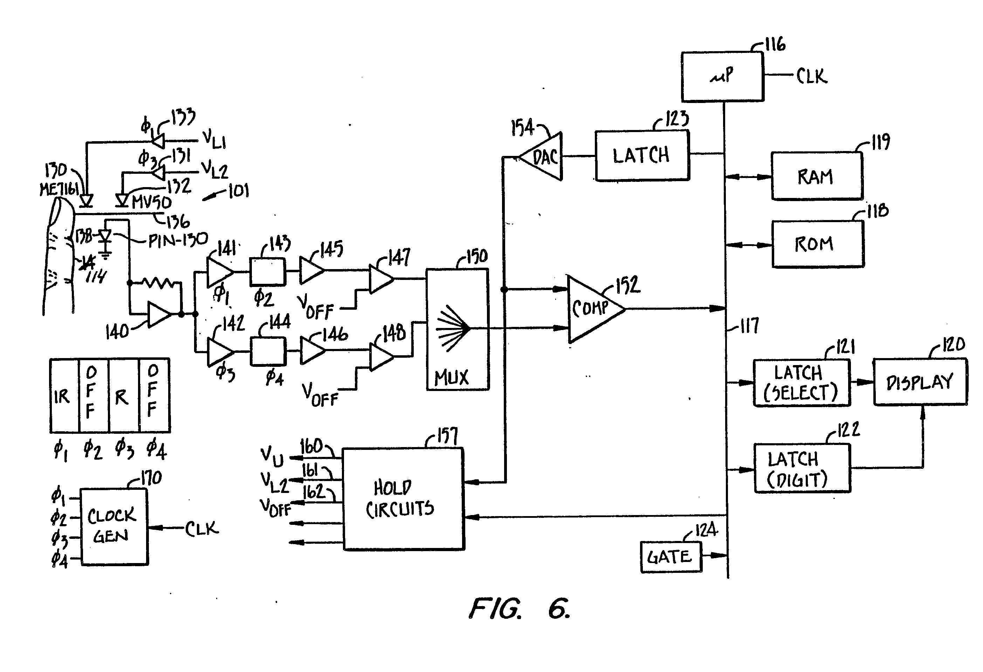

Referring to Fig. 6, the pulse oximeter of this invention is illustrated.

Conventional microprocessor 116 has a bus 117 extending therefrom. Bus 117 has connected thereto conventional ROM 118 and RAM 119. An LED display 120 is schematically illustrated having a select latch 121 and a digit designation latch 122.

Having set forth the more or less conventional portions of the microprocessor, attention will now be directed to the analog portions of the circuitry.

Finger 114 of a patient is illustrated with probe 101 having schematic detection circuitry. First light emitting diode 132 in the red range and a second light emitting diode 130 in the infrared range are sequentially pulsed to emit light in their respective frequencies by amplifiers 131, 133. Typically, LED 132 is in the 6600 angstrom range (660 nm) with LED 130 being in the 9400 angstrom range (940 nm).

It is necessary that all the light from the active light emitting diode go through the flesh in finger 114. Therefore, a light impervious barrier 136 is placed between photosensor 138 and finger 114. Barrier 136, terminating in contact with the flesh of finger 114, makes the path between the respective light emitting diodes 130, 132 and the light receiving diode 138 occur only through the flesh of finger 114.

Signal received from the respective light emitting diodes first passes through a pre-amplifier 140. This signal is thereafter amplified in parallel at amplifiers 141, 142. As amplified, the signal is passed in parallel from each amplifier through respective phase detectors 143, 144. Passage through respective low pass filters 145, 146 thereafter occurs. Amplification at offset amplifiers 147, 148 then takes place. The pulsatile component is passed to multiplexer 150.

Multiplexer 150 has output to a comparator 152. Comparator 152 is ramped in half steps by a 12 bit digital to analog converter (hereinafter DAC) 154. DAC 154 places a comparison signal divided in one part from 4096 parts with the comparator outputting to bus 117.

The reader will recognize that not all human fingers and appendages are the same. Specifically, the difference between the races, skin pigment, weight, age, maturity and other factors all can lead to different signals being sensed at photosensor 138, even though the frequency and intensity of the light signal output at each of the diodes 130, 132 is the same.

Accordingly, microprocessor 116 is programmed to receive signal from photosensor 138 within an optimum range. Utilizing a second operating phase of DAC 154, and communicating signal to a sample hold 157, the individual LED's 130, 132 are given voltage outputs 160, 161. These voltage outputs 160, 161 are adjusted so that in each case photosensor 138 looks at a signal well within the range of the DAC.

Clock 170 controls the sequential output of light from the light emitting diodes 130, 132 to a duty cycle of at least 1 in 4. This is schematically illustrated by signals φ1 through φ4. Reception of signal at detector 143 occurs during time periods φ1 and φ2 and reception of signal occurs at detector 144 during time periods φ3 and φ4.

It can be immediately realized that during respective time periods φ1, φ3 active signal from the light emitting diodes 130, 132 is being received. During the time periods φ2 and φ4, no signal and only noise is being received. As will hereinafter become apparent, by amplifying the negative signal before passage through the low pass filter, noise can be subtracted out utilizing the illustrated 1 in 4 duty cycle.

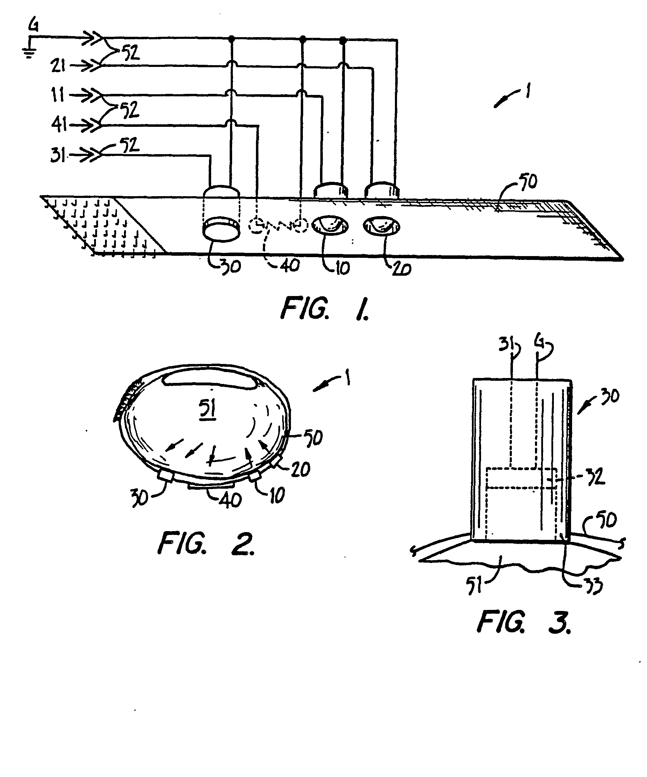

Figure 6 is a copy of Fig. 2 from European patent application No. 83304938 having been renumbered to avoid confusion with the context herewith. The above application is concerned with a pulse oximeter of the type wherein light of two different wavelengths is passed through human or animal body tissue, such as a finger, an ear, the nasal septum or the scalp, so as to be modulated by the pulsatile component of arterial blood therein, and thereby allowing indication of oxygen saturation, blood perfusion and heart rate. The level of incident light is continually adjusted for optimal detection of the pulsatile component, while permitting accommodation to variable attenuations due to skin color, flesh thickness and other invariants. At significant slope reversal of the pulsatile component to negative (indicating a wave maximum), wave form analysis of blood flow occurs. A quotient of the pulsatile component of light transmission over the constant component of light transmission is measured for each of two wavelengths by direct digital tracking. The respective quotients are thereafter converted to a ratio, which ratio may be thereafter fitted to a curve of independently derived of oxygen saturation. Calibration is disclosed by solving four unknowns at at least four differing saturations. An output of pulse rate, pulsatile flow and oxygen saturation is given. An incident light source duty cycle is chosen to be at least 1 in 4 so that noise, inevitably present in the signal, may be substantially eliminated and filtered.

Referring to Fig. 1 of the present application, a part-schematic, part-perspective view of the optical probe 1 is shown. A suitable length of adjustable, self-fastening tape 50 is provided, such as that sold under the trade mark VELCRO, obtainable from American Velcro, Inc. Incorporated into tape 50 at suitably spaced intervals are the electrical components of probe 1. Photoelectric sensor 30 is attached to the outside of tape 50 and protrudes slightly from the underside of tape 50.

Sensor 30 has ground wire G and lead wire 31.

Light emitting diode 10, typically emitting frequencies in the infrared range of the spectrum, is mounted to and pierces tape 50 in a similar manner to sensor 30 and at a distance from sensor 30 selected upon the basis of the typical appendage size expected to be encountered. LED 10 is connected to ground wire G and has input lead wire 11. Placed in proximity to LED 10 is a second LED 20, typically having wavelength emission characterisics in the red range of the spectrum. LED 20 attaches to ground wire G and has input lead wire 21.

Resistor 40 is shown mounted to tape 50 between sensor 30 and LED 10. However, the physical location of resistor 40 is not important and it may be mounted to probe 1 at any other convenient location. Resistor 40 has input lead wire 41 and is connected to ground wire G.

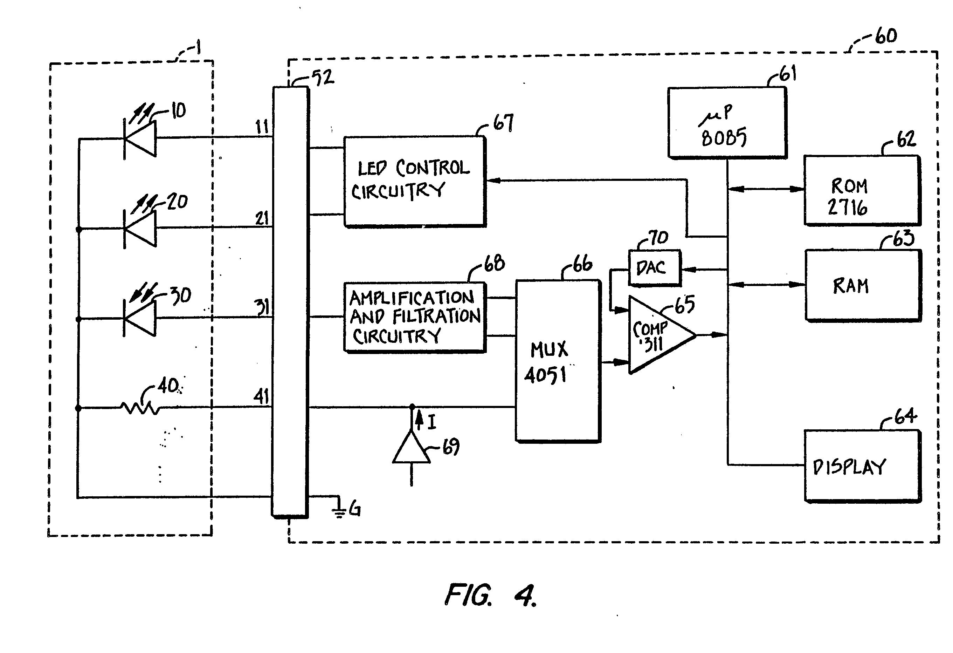

Wires G, 11, 21, 31, 41 lead to connector 52 so that probe 11 may be readily disconnected from the oximeter 60 (schematically illustrated in Fig. 4).

The probe 1 illustrated in Fig. 1 is designed for use in connection with an oximeter 60 designed to operate in conjunction with two LEDs 10, 20 sequentially transmitting light to a single sensor 30. However, the mechanism of the instant invention works equally well for oximeters requiring only a single LED and single or multiple photo sensors. Oximeters requiring more than two LEDs may be equally well accommodated by the probe of the present invention.

Fig. 2 is an end elevation of a typical finger 51 of a human patient. Finger 51 is encircled by probe 1 at its tip by overlapping the ends of self-connecting tape 50. Light emitted from LEDs 10, 20 enter the flesh of finger 51 and are subjected to diffusion and scattering. Sensor 30 picks up only light which has been diffused through the flesh of finger 51.

Fig. 3 is a detailed side elevation of sensor 30, showing the manner in which it is assured that no light emitted by LEDs 10, 20 is received by sensor 30 without first passing through finger 51. Sensor element 32 is recessed somewhat within metal cylinder wall 33 of the sensor housing. Since tape 50 presses sensor 30 directly against the skin of finger 51, it is readily seen that no light passes to sensor element 32 other than through the flesh of finger 51.

Probe 1 is constructed in the following manner: LED's 10, 20 are selected from batches of LED's with generally known wavelength characteristics. The exact wavelength characteristics of the specific LED's 10, 20 chosen are determined at this time through readily available metering means. Resistor 40 is then selected to have a resistance whose amount is exactly specified by a table made available to the factory technician for this purpose, of all possible wavelength combinations which may be expected to be encountered from the available supplies of LEDs. The following table is an example of how a single resistor 40 might be selected for any hypothetical combination of LED's 10, 20 in a case where each has only two possible wavelengths:

### TABLE A

| Resistor 40 | LED 10 | LED 20 |
|---|---|---|
| 150 ohms | 940 nM | 660 nM |
| 160 ohms | 950 nM | 660 nM |
| 170 ohms | 940 nM | 670 nM |
| 180 ohms | 950 nM | 670 nM |

A typical probe will have an infrared LED 10 of wavelength 940 nanometers and a red LED 20 of wavelength 660 nanometers. According to the above table, a probe having such wavelength characteristics will be supplied at the factory with a resistor 40 of one, and only one, resistance value, in this case shown to be 150 ohms.

The value in having such a unique known resistance incorporated into probe 1 is shown by reference to Fig. 4. Oximeter 60 contains a microprocessor 61, and a read only memory 62 and random access memory 63. Table A (the same table used for calibrating probe 1 at the factory) no matter how extensive, may be easily programmed into ROM 62 at the time oximeter 60 is fabricated. Current I from current source 69 is passed through resistor 40. The resulting voltage (per Ohm's law) is passed through multiplexer 66 through comparator 65, to microprocessor 61.

Microprocessor 61 may be programmed to calculate the resistance of resistor 40 and thereafter to look up the wavelengths of LED's 10, 20 from Table A in ROM 62. Microprocessor 61 is also programmed to itself recalibrate the optical comparison circuitry of oximeter 60 once the wavelengths of LEDs 10, 20 are known. By this means, it is not required to recalibrate by hand oximeter 60 for each new probe 1 nor, alternatively, to require that LEDs 10, 20 be of precisely standardized wavelengths.

The specific function and design of the circuitry schematically illustrated in Fig. 4 is seen as obvious when taken in combination with the general description of its function. The functions of microprocessors and read only memories are well known and understood and it is well within the capability of a person with ordinary skill in the art to design and program microprocessor 61 to calculate the resistance of resistor 40 and thereby obtain the wavelengths of LEDs 10, 20 from a simple lookup table in a ROM 62.

Probe 1 may be used with any number of prior art oximeters, the method of operation of which is well understood and beyond the scope of the teaching of the present invention. Basically, for each heart beat, fresh blood is pumped into the capillaries of finger 51, thereby causing a periodic increase and decrease in reflected light intensity observed by sensor 30. The oxygen saturation of hemoglobin in the pulsalite blood may be determined by the oximeter 60. For any known wavelength, there is a known extinction coefficient B. Given B and measuring the intensity of diffused light received by sensor 30 the oxygen saturation can be computed and displayed. In fact, the coefficients B of the various wavelengths of table A

can be substituted for the wavelengths directly when the table is programmed into ROM 62, thereby eliminating a computational step.

Microprocessor 61, through LED control circuitry 67, operates LEDs 10, 20. Light from LEDs 10, 20 results in current in sensor 30 which passes through amplification and filtration circuitry 68 to multiplexer 66. Comparator 65, in combination with a digital to analog converter 70 allows microprocessors 61 to determine oxygen saturation and/or pulse rate. Results are shown on display 64.

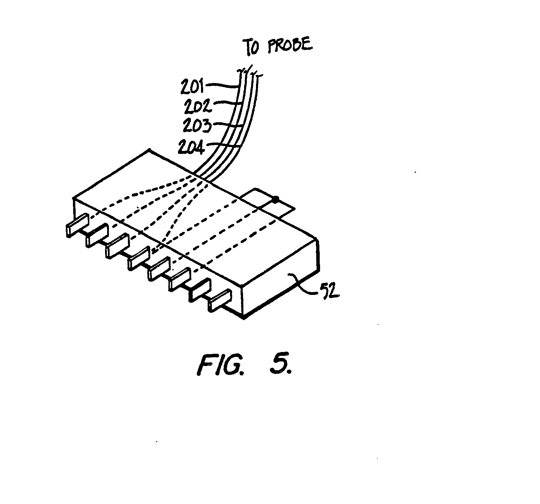

Referring to Fig. 5, an alternate way of coding a probe of this invention is illustrated. Specifically, an eight bit connector 52' similar to the connector 52 is illustrating having respective connectors 201, 202, 203 respectively communicating to light emitting diode 130, light emitting diode 132 and photodetector 138. Conductor 204 is illustrated providing the ground connection.

It will be noted that the eight bit connector has four empty channels. These channels can be provided to communicate the coded value of the probe.

For example, assuming that the connectors when provided with a common potential provide a positive binary value and when independent of any potential provide a negative value. Thus, the four conductors of plug 52, as illustrated in Fig. 5, would communicate the binary value 1100. Thus, communication of the resistance value of the connected probe would be possible by coding the connector to a value of 1 part in 16.

Those skilled in the art will appreciate that other binary connections could as well be made. For example, by expanding the number of connectors on the probe relatively large expansions can occur.

Those skilled in the art will realize that in determining the variable transmission of light in human flesh the frequency at which the flesh is integrated by a substantially monochromatic light source is critical. If the frequency varies the results of the instrument can be inaccurate with such variation. Simply stated, at different points in the spectral frequency, oxygenated hemoglobin and reduced hemoglobin transmit varying amounts of light.

Commercially produced light emitting diodes do have variation in their spectral frequency from diode to diode. Therefore if such commercially produced diodes are going to be used as replaceable probes in an instrument it has been found that provision must be made for a probe by probe calibration of the instrument. Thus, effectively disposable probes can be readily used even though they are affecting integration at differing frequencies from probe to probe.

Some comment can be made directed specifically at calibrating the disposable probe of the instrument herein. As a practical matter, the blood of a human is interrogated through the skin by light transmission utilizing red and infrared. The rate of change of constants in the infrared is relatively flat. Therefore a variance in the frequency of the infrared diode has little effect.

Not so in the red range. It has been found that the attenuation of light in oxygenated and unoxygenated hemoglobin has a rapidly changing slope in the red range. This being the case, it is of primary concern to calibrate in the particular instrument illustrated in the red range.

Those skilled in the art will realized that there are many ways in which change of instrument calibration can occur. Specifically, separate look-up tables can be generated for various grouped relationships. Alternately, and perhaps more productively, incremental alternation to the constants of curvature between the saturation level S and the ratio of quotients R of light transmission can be determined.

Although the foregoing invention is described in some detail by way of illustration and example for purpose of clarity of understanding, it is understood that certain changes and modifications may be practiced and equivalents employed within the scope of the appended claims. For example, two resistors may be used in place of one, each resistor coded to the wavelength of a separate LED. Other components could be used in place of resistors, e.g., capacitors or the like. Therefore, the above description and illustrations should not be construed as limiting the scope of the invention which is defined by the appended claims.

## Claims

1. An oximeter probe for use with an oximeter (60), said probe (1, 101) comprising:
   a first light emitting means (10, 132) emitting light having a first known wavelength value;
   means (30, 138) for sensing the light emitted by said first light emitting means (10, 132);
   means (52) for detachably wiring the probe (1, 101) to the oximeter (60) and for providing communication of electrical signals between the probe (1, 101) and the oximeter (60); and characterized by
   encoding means for providing signals to the oximeter (60) which are indicative of the known wavelength value of said first light emitting means (10, 132).

2. The probe of claim 1, further comprising a second light emitting means (20, 130) emitting light having a second known wavelength value;
   wherein said light sensing means (30, 138) also senses light emitted by said second light emitting means (20, 130); and
   wherein said encoding means further provides electrical signals to the oximeter (60) indicative of the known wavelength value of the light emitted by the second light emitting means (20, 130).

3. The probe of claim 1 or 2, wherein said encoding means comprises an electrical impedance element, the value of which is preselected to correlate with said known wavelength value.

4. The probe of claim 3, wherein said electrical impedance element is a resistor (40).

5. The probe of claim 4 wherein said signals which are indicative of said known wavelength value are voltage signals produced by passing a current through said resistor (40).

6. The probe of any of claims 1 to 3 wherein said means (52) for detachably wiring the probe (1, 101) to the oximeter (60) has associated therewith a plurality of connector pins, and wherein the presence or absence of electrical connections between certain of said connector pins comprises part of said encoding means.

7. An oximeter system comprising a probe according to any of claims 1 to 6 and an oximeter which is characterized by decoding means responsive to said encoded signals for selecting appropriate calibration coefficients for use in calculating oxygen saturation based upon the known wavelength of said first and/or second light emitting means (10, 132 and 20, 130, respectively).

8. The system according to claim 7, characterized in that said decoding means comprises a programmed microprocessor (61, 116).

**Patentansprüche**

1. Oximetriesonde für ein Oximeter (60), wobei die Sonde (1, 101) aufweist:

eine erste lichtemittierende Einrichtung (10, 132) zum Emittieren von Licht einer ersten bekannten Wellenlänge;

eine Einrichtung (30, 138) zum Detektieren des Lichtes, das von der ersten lichtemittierenden Einrichtung (10, 132) emittiert wird;

eine Einrichtung (52) zum lösbaren elektrischen Anschluß der Sonde (1, 101) an das Oximeter (60) und zum Übertragen von elektrischen Signalen zwischen der Sonde (1, 101) und dem Oximeter (60); gekennzeichnet durch

eine Codiereinrichtung zum Liefern von Signalen an das Oximeter (60), entsprechend dem bekannten Wellenlängenwert der ersten lichtemittierenden Einrichtung (10, 132).

2. Sonde nach Anspruch 1, die ferner eine zweite lichtemitterende Einrichtung (20, 130) aufweist, die Licht einer zweiten bekannten Wellenlänge aussendet;

wobei die Einrichtung (30, 138) zum Detektieren des Lichts auch Licht detektiert, das von der zweiten lichtemittierenden Einrichtung (20, 130) emittiert wird; und

wobei die Codiereinrichtungen ferner elektrische Signale an das Oximeter (60) entsprechend dem bekannten Wellenlängenwert des Lichtes liefert, das von der zweiten lichtemittierenden Einrichtung (20, 130) emittiert wird.

3. Sonde nach Anspruch 1 oder 2, wobei die Codiereinrichtung ein elektrisches Impedanzelement aufweist, dessen Wert so vorgewählt wird, daß er mit dem bekannten Wellenlängenwert korreliert.

4. Sonde nach Anspruch 3, wobei das elektrische Impedanzelement ein Widerstand (40) ist.

5. Sonde nach Anspruch 4, wobei die Signale, die für den bekannten Wellenlängenwert indikativ sind, Spannungssignale sind, die durch den Durchgang eines Stromes durch den Widerstand (40) erzeugt werden.

6. Sonde nach einem der Ansprüche 1 bis 3, wobei der Einrichtung (52) zum lösbaren elektrischen Anschluß der Sonde (1, 101) an das Oximeter (60) mehrere Verbindungsstifte zugeordnet sind, und wobei das Vorhandensein oder Nichtvorhandensein elektrischer Verbindungen zwischen bestimmten Verbindungsstiften ein Bestandteil der Codiereinrichtung ist.

7. Ein Oximetriesystem mit einer Sonde nach einem der Ansprüche 1 bis 6 und einem Oximeter, gekennzeichnet durch eine Dekodiereinrichtung, die auf die kodierten Signale zum Auswählen geeigneter Eichkoeffizienten für die Berechnung der Sauerstoffsättigung, basierend auf der bekannten Wellenlänge der ersten und/oder der zweiten lichtemittierenden Einrichtungen (10, 132 bzw. 20, 130), anspricht.

8. System nach Anspruch 7, dadurch gekennzeichnet, daß die Dekodiereinrichtung einen programmierten Mikroprozessor (61, 116) aufweist.

**Revendications**

1. Capteur oxymétrique à utiliser avec un oxymètre (60), ledit capteur (1, 101) comprenant des premiers moyens émettant de la lumière (10, 132) qui émettent de la lumière ayant une première valeur de longueur d'onde connue, des moyens (30, 138) pour détecter la lumière émise par lesdits premiers moyens émettant de la lumière (10, 132), des moyens (52) pour câbler de manière détachable le capteur (1, 101) à l'oxymètre (60) et pour réaliser une communication de signaux électriques entre le capteur (1, 101) et l'oxymètre (60), et caractérisé par des moyens d'encodage pour procurer à l'oxymètre (60) des signaux qui sont indicatifs de la valeur de longueur d'onde connue desdits premiers moyens émettant de la lumière (10, 132).

2. Capteur suivant la revendication 1, comprenant en outre des seconds moyens émettant une lumière (20, 130) qui émettent une lumière ayant une seconde valeur de longueur d'onde connue, caractérisé en ce que lesdits moyens de détection de lumière (30, 138) détectent aussi la lumière émise par lesdits seconds moyens émettant une lumière (20, 130) et en ce que lesdits moyens d'encodage procurent en outre à l'oxymètre (60) des signaux électriques indicatifs de la valeur de longueur d'onde connue de la lumière émise par les seconds moyens émettant une lumière (20, 130).

3. Capteur suivant l'une ou l'autre des revendications 1 et 2, caractérisé en ce que lesdits moyens d'encodage comprennent un élément à impédance électrique dont la valeur est présélectionnée afin de correspondre à ladite valeur de longueur d'onde connue.

4. Capteur suivant la revendication 3, caractérisé en ce que ledit élément à impédance électrique est une résistance (40).

5. Capteur suivant la revendication 4, caractérisé en ce que lesdits signaux qui sont indicatifs de ladite valeur de longueur d'onde connue sont des signaux de tension produits en faisant passer un courant à travers ladite résistance (40).

6. Capteur suivant l'une quelconque des revendications 1 à 3, caractérisé en ce que lesdits moyens (52) pour câbler de manière détachable la sonde (1, 101) à l'oxymètre (60) ont, associés avec cela, un certain nombre de broches de connecteurs et en ce que la présence ou l'absence de connexions électriques entre certaines desdites broches de connecteur comprend une partie desdits moyens d'encodage.

7. Système oxymétrique comprenant un capteur suivant l'une quelconque des revendications 1 à 6 et un oxymètre qui est caractérisé par des moyens de décodage sensibles auxdits signaux encodés afin de choisir des coéfficients de calibrage appropriés à utiliser dans le calcul d'une saturation en oxygène basée sur la longueur d'onde connue desdits premiers et/ou seconds moyens émettant de la lumière (10, 132 et 20, 130 respectivement).

8. Système suivant la revendication 7, caractérisé en ce que lesdits moyens de décodage comportent un microprocesseur programmé (61, 116).

FIG. 1.

FIG. 2.

FIG. 3.

FIG. 4.

FIG. 5.

FIG. 6.

EP 0 104 772 B1